# EUROPEAN PATENT APPLICATION

(11) **EP 2 815 704 A1**
(43) Date of publication of application: **24.12.2014**
(21) Application number: 14172684.4
(22) Date of filing: 17.06.2014
(51) Int. Cl.: A61B 17/072, A61B 19/00, A61B 17/00

(54) **Emergency retraction for electro-mechanical surgical devices and systems**

(30) Priority: 18.06.2013 US 201361836180 P; 23.05.2014 US 201414285782
(71) Applicant: Covidien LP, Mansfield, MA 02048 (US)
(72) Inventor: Ingmanson, Michael, Stratford, Connecticut 06614 (US); Snow, Joshua, Clinton, Connecticut 06413 (US); Wingardner, Thomas, North Haven, Connecticut 06473 (US); McCuen, David M., Stratford, Connecticut 06615 (US); Irka, Philip, Northford, Connecticut 06472 (US)
(74) Representative: Soames, Candida Jane

(57) **Abstract**

The present disclosure relates to an electromechanical surgical system (10) including a surgical instrument (100) having a controller (150 or 154), and an end effector (300) selectively and removably connectable to the surgical instrument, wherein the controller is configured to enter an emergency retraction mode (540, 640, 740) when at least one input element (124, 126, 128, 130) of the surgical system is incapable of providing control signals to the controller to operate a motor (166), wherein the emergency retraction mode activates the motor to withdraw a drive assembly (360) from any advanced position thereof.

## Description

### BACKGROUND

This application claims the benefit of and priority to U.S. Provisional Patent Application No. 61/836,180, filed June 18, 2013, the entire disclosure of which is incorporated by reference herein

### BACKGROUND

### 1. Technical Field

The present disclosure relates to surgical devices and/or systems, and more specifically, to electromechanical robotic and/or hand-held powered surgical devices and/or systems including an emergency retraction algorithm.

### 2. Background of Related Art

A number of surgical device manufacturers have developed product lines with proprietary drive systems for operating and/or manipulating electromechanical surgical devices. In many instances the electromechanical surgical devices include a reusable handle assembly, and disposable or single use end effectors. The end effectors are selectively connected to the handle assembly prior to use and then disconnected from the handle assembly following use in order to be disposed of or in some instances sterilized for re-use.

Many of these electromechanical surgical devices include complex drive components that utilize a variety of user interfaces that accept user inputs (e.g., controls, buttons, toggles, screens, switches, etc.) for controlling the devices as well as provide feedback to the user. To prevent actuation of drive mechanisms beyond mechanical limits, various switches and sensors are used to detect operational state of the surgical devices. Inclusion of multiple switches and/or sensors in the devices as well as end effectors presents various problems. In addition, cost or other considerations prevent the use of such devices. Accordingly, there is a need for systems and apparatuses having safety mechanisms that can detect mechanical limits without relying on multiple mechanical limit sensors and/or switches disposed throughout the surgical device.

Robotic systems for performing minimally invasive surgery are also known. In WO 2000/51486, the entire contents of which are incorporated herein by reference, a system is disclosed in which surgical instruments are remotely controlled.

Additionally, electromechanical surgical devices offer distinct advantages over purely mechanical devices. However, such electromechanical surgical devices are susceptible to previously unconsidered failure modes. For example, one such failure mode is the malfunction of any one or all of the user inputs, identified above, or otherwise contemplated or implemented. Such a failure could result in the electromechanical surgical device being rendered non-operational at a time when at least minimum levels of functionality are critical.

Accordingly, a need exists for methods, in the form of algorithms, and associated software and hardware implementing the methods which reduce risks associated with such failures of electromechanical surgical devices.

### SUMMARY

The present disclosure relates to electromechanical robotic and/or hand-held powered surgical devices and/or systems including an emergency retraction algorithm.

According to an aspect of the present disclosure, an electromechanical surgical system is provided comprises a hand-held surgical instrument including a handle housing; a motor disposed within the handle housing; a controller disposed within the handle housing and being in electrical communication with the motor; a battery selectively, removably insertable into the handle housing and being in electrical communication with at least one of the motor and the controller when disposed within the handle housing; and at least one input element supported on the handle housing and actuatable by a user to send control signals to the controller to operate the motor. The surgical system also comprises an end effector selectively and removably connectable to the surgical instrument, the end effector including a jaw assembly having a staple cartridge containing a plurality of staples and an anvil to form the plurality of staples upon firing; and a drive assembly at least partially located within the jaw assembly and operatively connectable to the motor when the end effector is connected to the surgical instrument for actuation by the motor. The controller is configured to enter an emergency retraction mode when the at least one input element is incapable of providing control signals to the controller to operate the motor, wherein the emergency retraction mode activates the motor to withdraw the drive assembly from any advanced position thereof.

The emergency retraction mode may be entered upon a removal and a re-insertion of the battery into the handle housing.

Alternatively, the emergency retraction mode may be entered by activating a switch, optionally protected by additional switch sequences or interlocks or by entering a specialized sequence of control buttons normally used for other purposes.

The emergency retraction mode may also be actuated by encountering an end effector with a readable memory indicating the end effector was previously used or associated with a previous an electromechanical surgical system.

In use, during the emergency retraction mode, the controller may undergo a re-boot, whereby the controller detects that the end effector is connected to the surgical instrument.

In the emergency retraction mode, the drive assembly may be automatically retracted.

In use, retraction of the drive assembly to a fully retracted position, may open the jaw assembly.

In use, in the emergency retraction mode the controller may disable all remaining functions of the surgical system.

The emergency retraction mode may be entered upon a removal of the battery into the handle housing, then a pressing and holding of a safety button, and then a re-insertion of the battery into the handle housing, wherein the safety button is supported on the handle housing.

In use, during the emergency retraction mode, the controller may undergo a re-boot, whereby the controller detects that the end effector is connected to the surgical instrument.

In use, following retraction of the drive assembly, when the safety button is released, the controller may not automatically re-advance the drive assembly.

In use, following a release of the safety button, and following a re-pressing and holding of the safety button, in the emergency retraction mode, the controller may activate the motor to re-close the jaw assembly.

In use, in the emergency retraction mode the controller may disable all remaining functions of the surgical system with the exception of an articulation function.

In use, in the emergency retraction mode, the surgical instrument may be operable to articulate the end effector.

According to another aspect of the present disclosure, a method for controlling an electromechanical surgical system that includes a hand-held surgical instrument selectively and removably supporting an end effector, is provided. The surgical instrument includes a motor, a controller in electrical communication with the motor, a battery in electrical communication with at least one of the motor and the controller, and at least one input element actuatable by a user to send control signals to the controller to operate the motor; and wherein the end effector includes a jaw assembly, a drive assembly at least partially located within the jaw assembly and operatively connectable to the motor when the end effector is connected to the surgical instrument for actuation by the motor.

The method comprises the steps of monitoring the at least one input element; when the at least one input element is incapable of providing control signals to the controller, entering an emergency retraction mode; activating the motor to withdraw the drive assembly from any advanced position thereof.

The method may further comprise the step of entering the emergency retraction mode upon a removal and a re-insertion of the battery into the surgical instrument.

The method may further comprise the steps of wherein during the emergency retraction mode, undergoing a re-boot of the controller; and detecting, by the controller, that the end effector is connected to the surgical instrument.

The method may further comprise the step of retracting the drive assembly automatically while in the emergency retraction mode.

The method may further comprise the step of opening the jaw assembly concomitantly with the retraction of the drive assembly to a fully retracted position.

The method may further comprise step of the controller disabling all remaining functions of the surgical system when in the emergency retraction mode.

The method may further comprise the steps of entering the emergency retraction mode upon a removal and a re-insertion of the battery into the surgical instrument; thereafter, pressing and holding of a safety button supported on the surgical instrument; and thereafter, reinserting the battery into the surgical instrument.

The method may further comprise the steps of wherein during the emergency retraction mode, undergoing a re-boot of the controller; and detecting, by the controller, that the end effector is connected to the surgical instrument.

The method may further comprise the step of retracting the drive assembly automatically while in the emergency retraction mode.

The method may further comprise the step of opening the jaw assembly concomitantly with the retraction of the drive assembly to a fully retracted position.

The method may further comprise the step of, following retraction of the drive assembly, and following a release of the safety button, the controller does not automatically re-advance the drive assembly.

The method may further comprise the step of, following a release of the safety button, and following a re-pressing and holding of the safety button, in the emergency retraction mode, the controller activates the motor to re-close the jaw assembly.

The method may further comprise the step of, while in the emergency retraction mode, disabling all remaining functions of the surgical system, by the controller, with the exception of an articulation function.

The method may further comprise the step of articulating the end effector while in the emergency retraction mode.

### BRIEF DESCRIPTION OF THE DRAWINGS

Embodiments of the present disclosure are described herein with reference to the accompanying drawings, wherein:
FIG. 1 is a perspective, disassembled view of an electromechanical surgical system including a surgical instrument, an adapter, and an end effector, according to the present disclosure;
FIG. 2 is a perspective view of the surgical instrument of FIG. 1, according to the present disclosure;
FIG. 3 is perspective, exploded view of the surgical instrument of FIG. 1, according to the present disclosure;
FIG. 4 is a perspective view of a battery of the surgical instrument of FIG. 1, according to the present disclosure;
FIG. 5 is a top, partially-disassembled view of the surgical instrument of FIG. 1, according to the present disclosure;
FIG. 6 is a front, perspective view of the surgical instrument of FIG. 1 with the adapter separated therefrom, according to the present disclosure;
FIG. 7 is a side, cross-sectional view of the surgical instrument of FIG. 1, as taken through 7-7 of FIG. 2, according to the present disclosure;
FIG. 8 is a top, cross-sectional view of the surgical instrument of FIG. 1, as taken through 8-8 of FIG. 2, according to the present disclosure;
FIG. 9 is a perspective, exploded view of a end effector of FIG. 1, according to the present disclosure;
FIG. 10 is a schematic diagram of the surgical instrument of FIG. 1 according to the present disclosure;
FIG. 11 is a flow chart of an emergency retraction algorithm for the electromechanical surgical system, according to an embodiment of the present disclosure;
FIG. 12 is a flow chart of an emergency retraction algorithm for the electromechanical surgical system, according to another embodiment of the present disclosure; and
FIG. 13 is a flow chart of an emergency retraction algorithm for the electromechanical surgical system, according to yet another embodiment of the present disclosure.

### DETAILED DESCRIPTION OF EMBODIMENTS

Embodiments of the presently disclosed electromechanical surgical system, surgical devices, and adapter assemblies for surgical devices and/or handle assemblies are described in detail with reference to the drawings, in which like reference numerals designate identical or corresponding elements in each of the several views. As used herein the term "distal" refers to that portion of the electromechanical surgical system, the adapter assembly or the surgical device, or components thereof, farther from the user, while the term "proximal" refers to that portion of the electromechanical surgical system, the adapter assembly or the surgical device, or components thereof, closer to the user.

A surgical system, in accordance with an embodiment of the present disclosure, is generally designated as 10, and is in the form of a powered hand held electromechanical instrument configured for selective attachment thereto of a plurality of different end effectors that are each configured for actuation and manipulation by the powered hand held electromechanical surgical instrument.

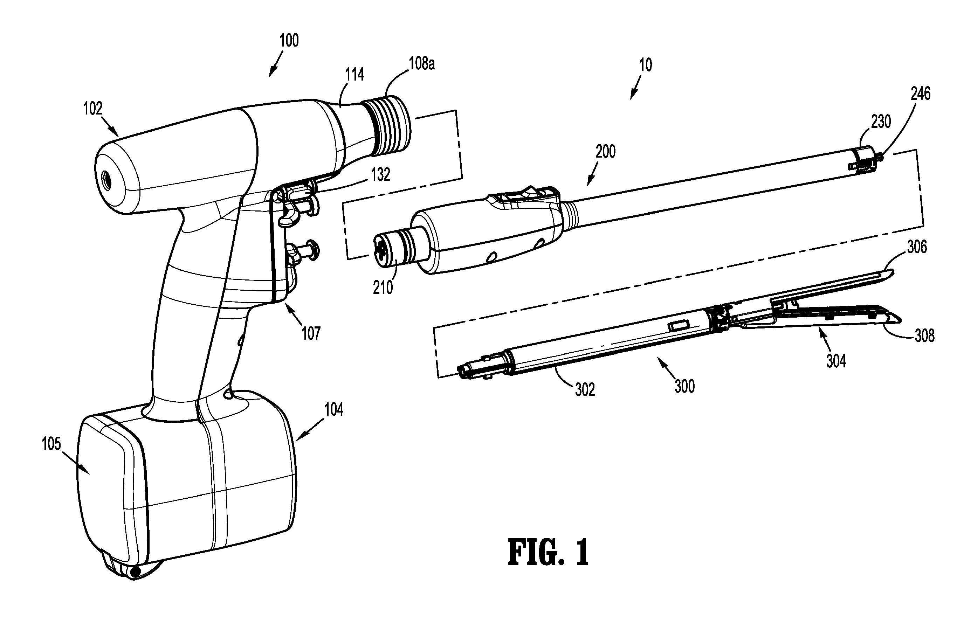

As illustrated in FIG. 1, surgical instrument 100 is configured for selective connection with an adapter 200, and, in turn, adapter 200 is configured for selective connection with an end effector or single use loading unit 300.

As illustrated in FIGS. 1-3, surgical instrument 100 includes a handle housing 102 having a lower housing portion 104, an intermediate housing portion 106 extending from and/or supported on lower housing portion 104, and an upper housing portion 108 extending from and/or supported on intermediate housing portion 106. Intermediate housing portion 106 and upper housing portion 108 are separated into a distal half-section 110a that is integrally formed with and extending from the lower portion 104, and a proximal half-section 110b connectable to distal half-section 110a by a plurality of fasteners. When joined, distal and proximal half-sections 110a, 110b define a handle housing 102 having a cavity 102a therein in which a circuit board 150 and a drive mechanism 160 is situated.

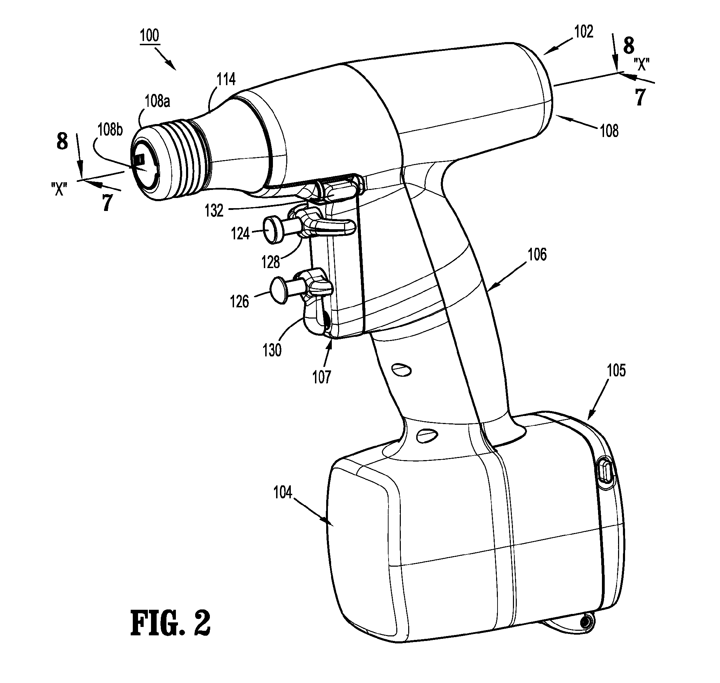

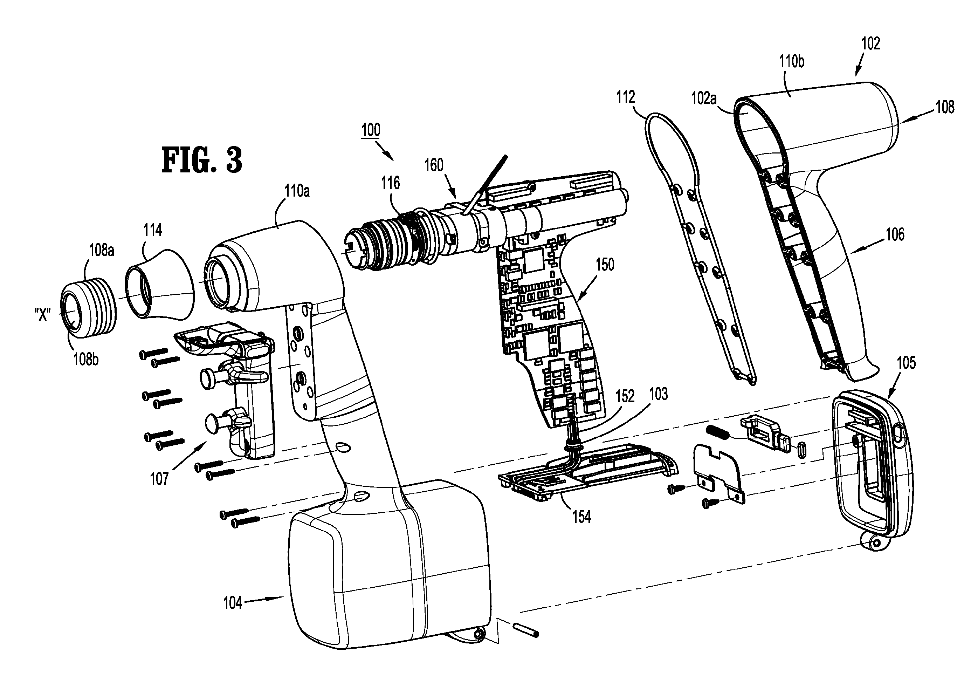

Distal and proximal half-sections 110a, 110b are divided along a plane that traverses a longitudinal axis "X" of upper housing portion 108, as seen in FIGS. 2 and 3. Handle housing 102 includes a gasket 112 extending completely around a rim of distal half-section and/or proximal half-section 110a, 110b and being interposed between distal half-section 110a and proximal half-section 110b. Gasket 112 seals the perimeter of distal half-section 110a and proximal half-section 110b. Gasket 112 functions to establish an air-tight seal between distal half-section 110a and proximal half-section 110b such that circuit board 150 and drive mechanism 160 are protected from sterilization and/or cleaning procedures.

In this manner, the cavity 102a of handle housing 102 is sealed along the perimeter of distal half-section 110a and proximal half-section 110b yet is configured to enable easier, more efficient assembly of circuit board 150 and a drive mechanism 160 in handle housing 102.

Intermediate housing portion 106 of handle housing 102 provides a housing in which circuit board 150 is situated. Circuit board 150 is configured to control the various operations of surgical instrument 100, as will be set forth in additional detail below.

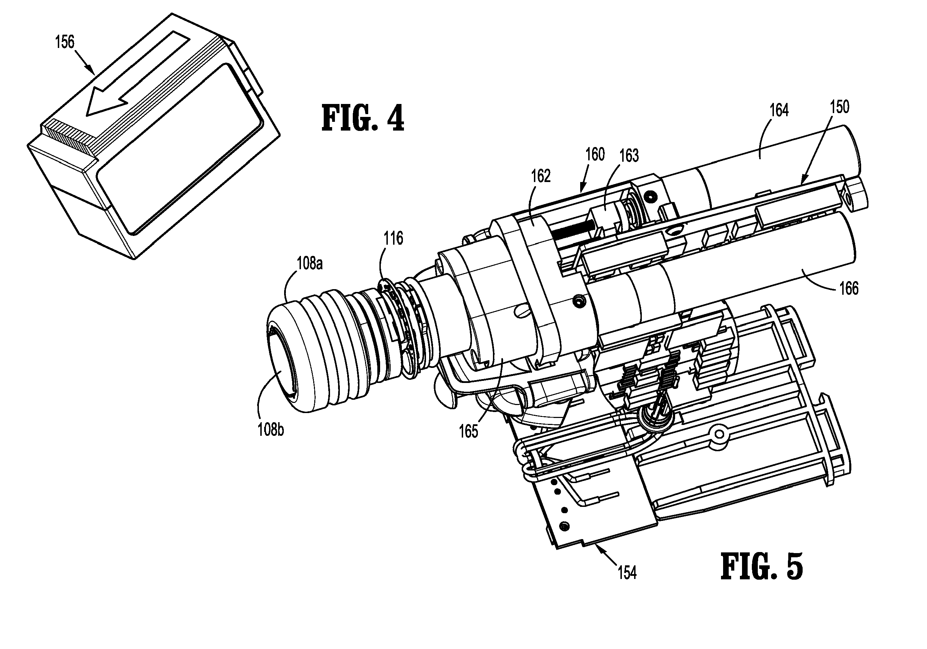

Lower housing portion 104 of surgical instrument 100 defines an aperture (not shown) formed in an upper surface thereof and which is located beneath or within intermediate housing portion 106. The aperture of lower housing portion 104 provides a passage through which wires 152 pass to electrically interconnect electrical components (a battery 156, as illustrated in FIG. 4, a circuit board 154, as illustrated in FIG. 3, etc.) situated in lower housing portion 104 with electrical components (circuit board 150, drive mechanism 160, etc.) situated in intermediate housing portion 106 and/or upper housing portion 108.

Handle housing 102 includes a gasket 103 disposed within the aperture of lower housing portion 104 thereby plugging or sealing the aperture of lower housing portion 104 while allowing wires 152 to pass therethrough. Gasket 103 functions to establish an air-tight seal between lower housing portion 106 and intermediate housing portion 108 such that circuit board 150 and drive mechanism 160 are protected from sterilization and/or cleaning procedures.

As shown, lower housing portion 104 of handle housing 102 provides a housing in which a rechargeable battery 156, is removably situated. Battery 156 is configured to supply power to any of the electrical components of surgical instrument 100. Lower housing portion 104 defines a cavity (not shown) into which battery 156 is inserted. Lower housing portion 104 includes a door 105 pivotally connected thereto for closing cavity of lower housing portion 104 and retaining battery 156 therein.

With reference to FIGS. 3 and 5, distal half-section 110a of upper housing portion 108 defines a nose or connecting portion 108a. A nose cone 114 is supported on nose portion 108a of upper housing portion 108. Nose cone 114 is fabricated from a transparent material. An illumination member 116 is disposed within nose cone 114 such that illumination member 116 is visible therethrough. Illumination member 116 is may be a light emitting diode printed circuit board (LED PCB). Illumination member 116 is configured to illuminate multiple colors with a specific color pattern being associated with a unique discrete event.

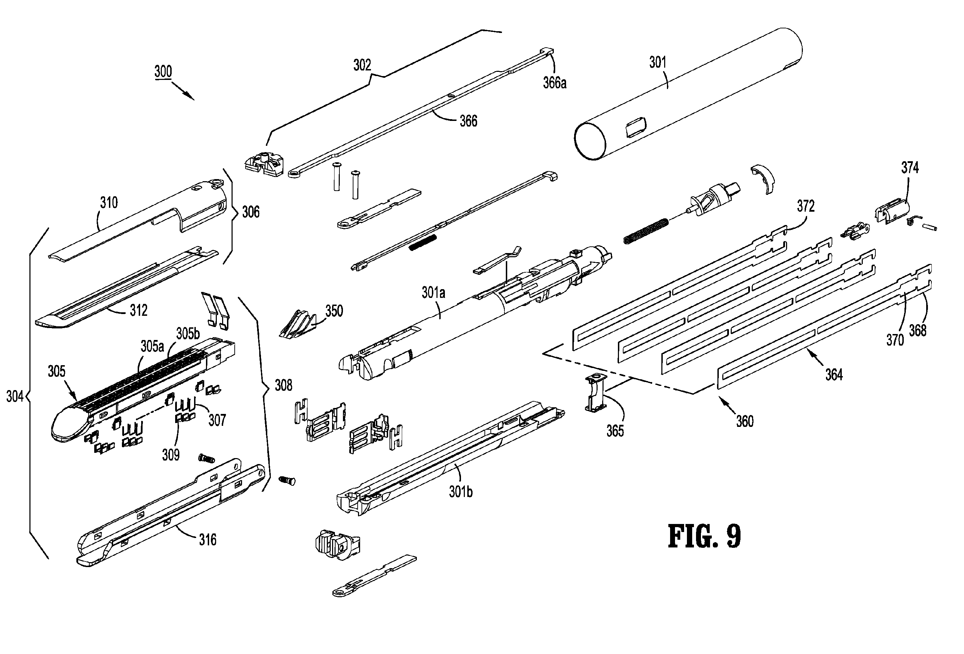

Upper housing portion 108 of handle housing 102 provides a housing in which drive mechanism 160 is situated. As illustrated in FIG. 5, drive mechanism 160 is configured to drive shafts and/or gear components in order to perform the various operations of surgical instrument 100. In particular, drive mechanism 160 is configured to drive shafts and/or gear components in order to selectively move tool assembly 304 of end effector 300 (see FIGS. 1 and 9) relative to proximal body portion 302 of end effector 300, to rotate end effector 300 about a longitudinal axis "X" (see FIG. 2) relative to handle housing 102, to move anvil assembly 306 relative to cartridge assembly 308 of end effector 300, and/or to fire a stapling and cutting cartridge within cartridge assembly 308 of end effector 300.

The drive mechanism 160 includes a selector gearbox assembly 162 that is located immediately proximal relative to adapter 200. Proximal to the selector gearbox assembly 162 is a function selection module 163 having a first motor 164 that functions to selectively move gear elements within the selector gearbox assembly 162 into engagement with an input drive component 165 having a second motor 166.

As illustrated in FIGS. 1-4, and as mentioned above, distal half-section 110a of upper housing portion 108 defines a connecting portion 108a configured to accept a corresponding drive coupling assembly 210 of adapter 200.

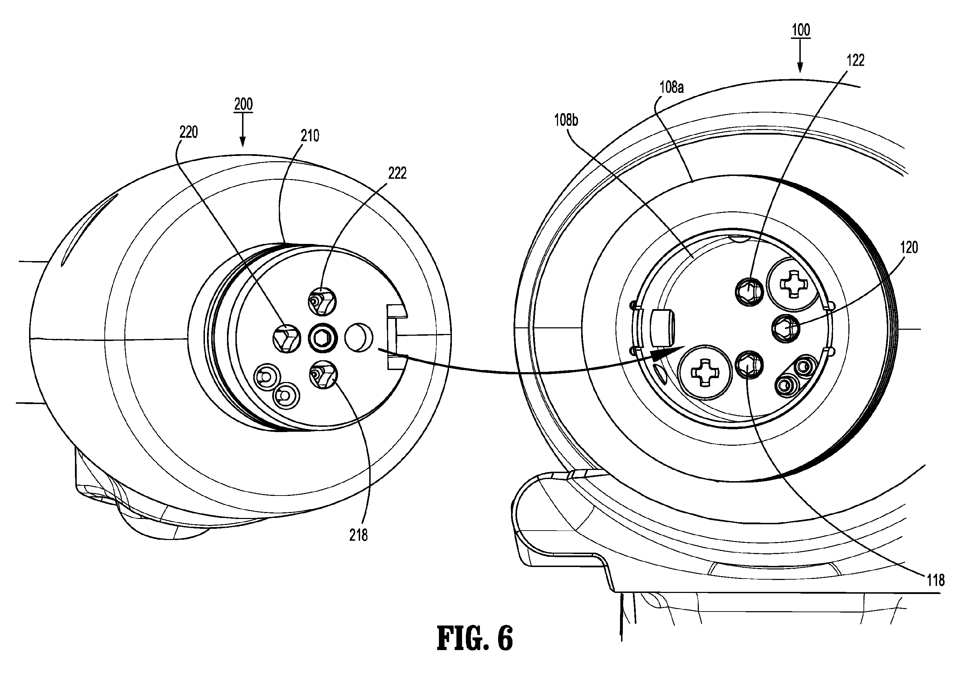

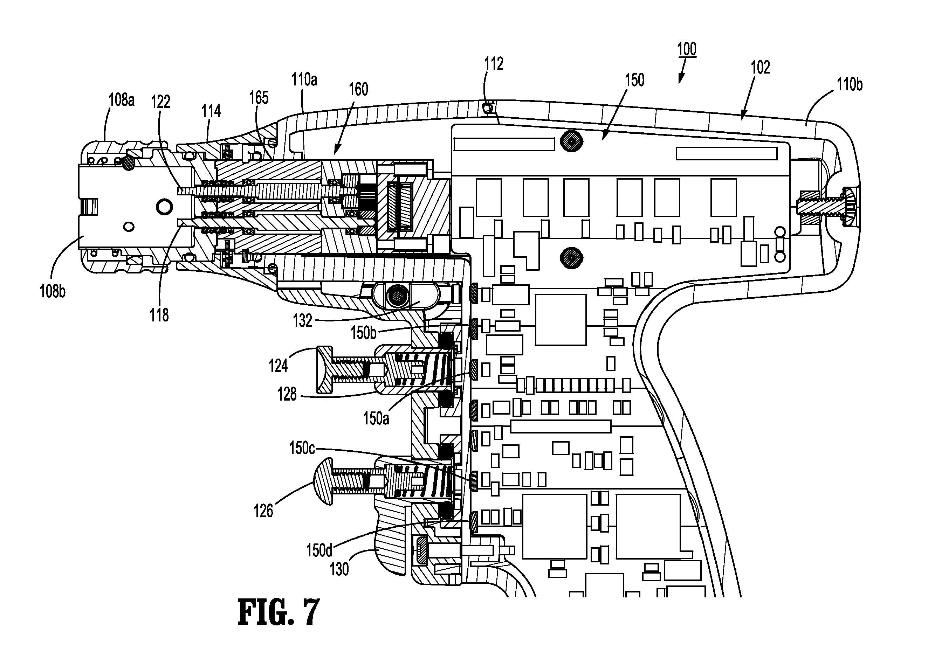

As illustrated in FIGS. 6-8, connecting portion 108a of surgical instrument 100 has a cylindrical recess 108b that receives a drive coupling assembly 210 of adapter 200 when adapter 200 is mated to surgical instrument 100. Connecting portion 108a houses three rotatable drive connectors 118, 120, 122.

When adapter 200 is mated to surgical instrument 100, each of rotatable drive connectors 118, 120, 122 of surgical instrument 100 couples with a corresponding rotatable connector sleeve 218, 220, 222 of adapter 200, as shown in FIG. 6. In this regard, the interface between corresponding first drive connector 118 and first connector sleeve 218, the interface between corresponding second drive connector 120 and second connector sleeve 220, and the interface between corresponding third drive connector 122 and third connector sleeve 222 are keyed such that rotation of each of drive connectors 118, 120, 122 of surgical instrument 100 causes a corresponding rotation of the corresponding connector sleeve 218, 220, 222 of adapter 200.

The mating of drive connectors 118, 120, 122 of surgical instrument 100 with connector sleeves 218, 220, 222 of adapter 200 allows rotational forces to be independently transmitted via each of the three respective connector interfaces. The drive connectors 118, 120, 122 of surgical instrument 100 are configured to be independently rotated by drive mechanism 160. In this regard, the function selection module 163 of drive mechanism 160 selects which drive connector or connectors 118, 120, 122 of surgical instrument 100 is to be driven by the input drive component 165 of drive mechanism 160.

Since each of drive connectors 118, 120, 122 of surgical instrument 100 has a keyed and/or substantially non-rotatable interface with respective connector sleeves 218, 220, 222 of adapter 200, when adapter 200 is coupled to surgical instrument 100, rotational force(s) are selectively transferred from drive mechanism 160 of surgical instrument 100 to adapter 200.

The selective rotation of drive connector(s) 118, 120 and/or 122 of surgical instrument 100 allows surgical instrument 100 to selectively actuate different functions of end effector 300. As will be discussed in greater detail below, selective and independent rotation of first drive connector 118 of surgical instrument 100 corresponds to the selective and independent opening and closing of tool assembly 304 of end effector 300, and driving of a stapling/cutting component of tool assembly 304 of end effector 300. Also, the selective and independent rotation of second drive connector 120 of surgical instrument 100 corresponds to the selective and independent articulation of tool assembly 304 of end effector 300 transverse to longitudinal axis "X" (see FIG. 2). Additionally, the selective and independent rotation of third drive connector 122 of surgical instrument 100 corresponds to the selective and independent rotation of end effector 300 about longitudinal axis "X" (see FIG. 2) relative to handle housing 102 of surgical instrument 100.

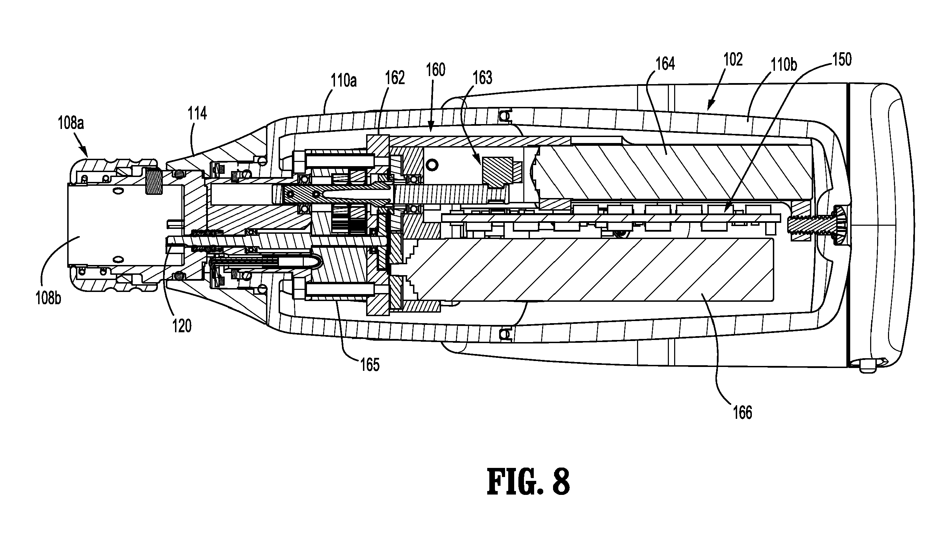

As mentioned above and as illustrated in FIGS. 5 and 8, drive mechanism 160 includes a selector gearbox assembly 162; and a function selection module 163, located proximal to the selector gearbox assembly 162, that functions to selectively move gear elements within the selector gearbox assembly 162 into engagement with second motor 166. Thus, drive mechanism 160 selectively drives one of drive connectors 118, 120, 122 of surgical instrument 100 at a given time.

As illustrated in FIGS. 1-3, handle housing 102 supports a control assembly 107 on a distal surface or side of intermediate housing portion 108. The control assembly 107 is a fully-functional mechanical subassembly that can be assembled and tested separately from the rest of the instrument 100 prior to coupling thereto.

Control assembly 107, in cooperation with intermediate housing portion 108, supports a pair of finger-actuated control buttons 124, 126 and a pair rocker devices 128, 130 within a housing 107a. The control buttons 124, 126 are coupled to extension shafts 125, 127 respectively. In particular, control assembly 107 defines an upper aperture 124a for slidably receiving the extension shaft 125, and a lower aperture 126a for slidably receiving the extension shaft 127.

As seen in FIGS. 1, 2 and 7, surgical instrument 100 includes a fire button or safety switch 132 supported between intermediate housing portion 108 and upper housing portion, and situated above trigger housing 107. In use, tool assembly 304 of end effector 300 is actuated between opened and closed conditions as needed and/or desired. In order to fire end effector 300, to expel fasteners therefrom when tool assembly 304 of end effector 300 is in a closed condition, safety switch 132 is depressed thereby instructing surgical device 100 that end effector 300 is ready to expel fasteners therefrom.

Reference may be made to U.S. Provisional Patent Application Serial No. 61/654,191, filed on June 1, 2012, entitled "Hand Held Surgical Handle Assembly, Surgical Adapters for Use Between Surgical Handle Assembly and Surgical End Effectors, and Methods of Use," the entire content of which is incorporated herein by reference, for a detailed discussion of the construction and operation of features and components of surgical instrument 100 that are not explicitly described herein.

Referring to FIG. 9, drive assembly 360 of end effector 300 includes a flexible drive shaft 364 having a distal end which is secured to a dynamic drive beam 365, and a proximal engagement section 368. Engagement section 368 includes a stepped portion defining a shoulder 370. A proximal end of engagement section 368 includes diametrically opposed inwardly extending fingers 372. Fingers 372 engage a hollow drive member 374 to fixedly secure drive member 374 to the proximal end of shaft 364. Drive member 374 defines a proximal porthole which receives a connection member of drive tube 246 (FIG. 1) of adapter 200 when end effector 300 is attached to distal coupling 230 of adapter 200.

When drive assembly 360 is advanced distally within tool assembly 304, an upper beam of drive beam 365 moves within a channel defined between anvil plate 312 and anvil cover 310 and a lower beam moves within a channel of the staple cartridge 305 and over the exterior surface of carrier 316 to close tool assembly 304 and fire staples therefrom.

Proximal body portion 302 of end effector 300 includes a sheath or outer tube 301 enclosing an upper housing portion 301a and a lower housing portion 301b. The housing portions 301a and 301b enclose an articulation link 366 having a hooked proximal end 366a which extends from a proximal end of end effector 300. Hooked proximal end 366a of articulation link 366 engages a coupling hook (not shown) of adapter 200 when end effector 300 is secured to distal housing 232 of adapter 200. When drive bar (not shown) of adapter 200 is advanced or retracted as described above, articulation link 366 of end effector 300 is advanced or retracted within end effector 300 to pivot tool assembly 304 in relation to a distal end of proximal body portion 302.

As illustrated in FIG. 9, cartridge assembly 308 of tool assembly 304 includes a staple cartridge 305 supportable in carrier 316. Staple cartridge 305 defines a central longitudinal slot 305a, and three linear rows of staple retention slots 305b positioned on each side of longitudinal slot 305a. Each of staple retention slots 305b receives a single staple 307 and a portion of a staple pusher 309. During operation of instrument 100, drive assembly 360 abuts an actuation sled 350 and pushes actuation sled 350 through cartridge 305. As the actuation sled moves through cartridge 305, cam wedges of the actuation sled 350 sequentially engage staple pushers 309 to move staple pushers 309 vertically within staple retention slots 305b and sequentially eject a single staple 307 therefrom for formation against anvil plate 312.

The end effector 300 may also include one or more mechanical lockout mechanisms, such as those described in commonly-owned U.S. Patent Nos. 5,071,052; 5,397,046; 5,413,267; 5,415,335; 5,715,988; 5,718,359; and 6,109,500, the entire contents of all of which are incorporated by reference herein. Reference may also be made to U.S. Patent Publication No. 2009/0314821, filed on August 31, 2009, entitled "TOOL ASSEMBLY FOR A SURGICAL STAPLING DEVICE" for a detailed discussion of the construction and operation of end effector 300.

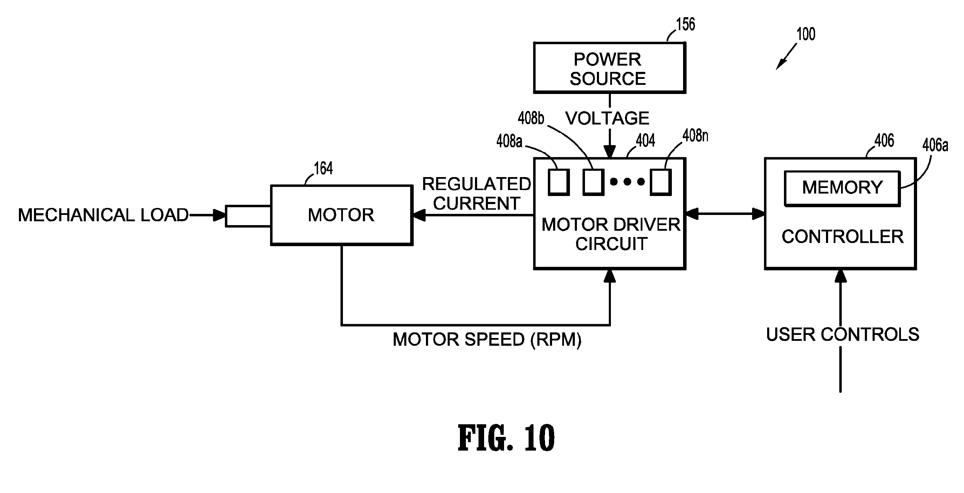

Another embodiment of the instrument 100 is shown in FIG. 10. The instrument 100 includes the motor 164. The motor 164 may be any electrical motor configured to actuate one or more drives (e.g., rotatable drive connectors 118, 120, 122 of FIG. 6). The motor 164 is coupled to the battery 156, which may be a DC battery (e.g., rechargeable lead-based, nickel-based, lithium-ion based, battery etc.), an AC/DC transformer, or any other power source suitable for providing electrical energy to the motor 164.

The battery 156 and the motor 164 are coupled to a motor driver circuit 404 disposed on the circuit board 154 which controls the operation of the motor 164 including the flow of electrical energy from the battery 156 to the motor 164. The instrument 100 may use multiple motors 164 and motor driver circuits 404 or a transmission associated with a motor 100 (all not shown) to drive various functions of the instrument. The driver circuit 404 includes a plurality of sensors 408a, 408b, 408n configured to measure operational states of the motor 164 and the battery 156. The sensors 408a-n may include voltage sensors, current sensors, temperature sensors, telemetry sensors, optical sensors, and combinations thereof. The sensors 408a-408n may measure voltage, current, and other electrical properties of the electrical energy supplied by the battery 156. The sensors 408a-408n may also measure rotational speed as revolutions per minute (RPM), torque, temperature, current draw, and other operational properties of the motor 164. RPM may be determined by measuring the rotation of the motor 164. Position of various drive shafts (e.g., rotatable drive connectors 118, 120, 122 of FIG. 6) may be determined by using various linear sensors disposed in or in proximity to the shafts or extrapolated from the RPM measurements. In embodiments, torque may be calculated based on the regulated current draw of the motor 164 at a constant RPM. In further embodiments, the driver circuit 404 and/or the controller 406 may measure time and process the above-described values as a function thereof, including integration and/or differentiation, e.g., to determine the change in the measured values and the like.

The driver circuit 404 is also coupled to a controller 406, which may be any suitable logic control circuit adapted to perform the calculations and/or operate according to a set of instructions described in further detail below. The controller 406 may include a central processing unit operably connected to a memory which may include transitory type memory (e.g., RAM) and/or non-transitory type memory (e.g., flash media, disk media, etc.). The controller 406 includes a plurality of inputs and outputs for interfacing with the driver circuit 404. In particular, the controller 406 receives measured sensor signals from the driver circuit 404 regarding operational status of the motor 164 and the battery 156 and, in turn, outputs control signals to the driver circuit 404 to control the operation of the motor 164 based on the sensor readings and specific algorithm instructions, which are discussed in more detail below. The controller 406 is also configured to accept a plurality of user inputs from a user interface (e.g., switches, buttons, touch screen, etc. of the control assembly 107 coupled to the controller 406).

The present disclosure provides for an apparatus and method for controlling the instrument 100 or any other powered surgical instrument, including, but not limited to, linear powered staplers, circular or arcuate powered staplers, clip appliers, graspers, electrosurgical sealing forceps, rotary tissue blending devices, and the like.

Specifically, according to the present disclosure, algorithms are provided for controlling surgical instrument 100 and/or surgical system 10 in the event that an input element (i.e., control buttons 124, 126, rocker devices 128, 130, other buttons, switches, touch screens, controls, toggles, etc.) should malfunction or otherwise become inoperable or non-responsive during a surgical procedure, i.e., following initial actuation of surgical instrument 100 and/or surgical system 10. In such a situation, it is imperative that surgical instrument 100 and/or surgical system 10 be provided with an emergency retraction algorithm to overcome any malfunction, inoperability or non-responsiveness of any input element of surgical instrument 100 and/or surgical system 10.

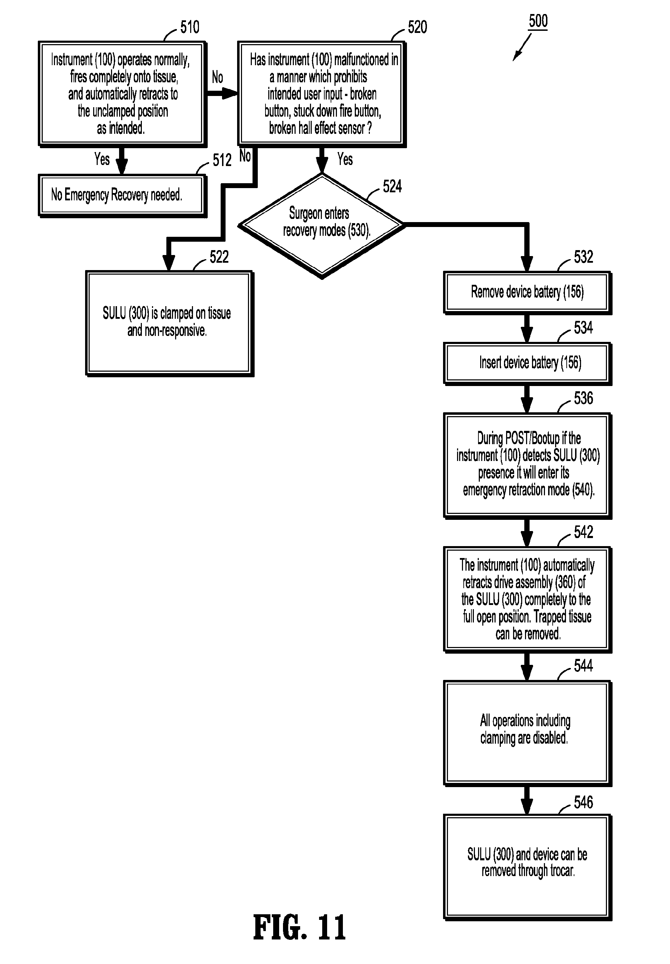

As seen in FIG. 11, a flow chart of an algorithm, in accordance with a first aspect of the present disclosure, is generally designated as 500. Computer code or instructions implementing algorithm 500 may be retained locally in circuit board 150 or circuit board 154 of surgical instrument 100, in a memory device (not shown) of end effector 300, or in a remote computer system (not shown) which is in wired or wireless communication with surgical instrument 100 and/or surgical system 10.

In use, during operation of surgical system 10, sensors and the like monitor the operation of surgical instrument 100 and/or end effector 300. As seen in the flow chart of FIG. 11, if, at step 510, surgical instrument 100 operates normally (i.e., end effector 300 fires completely into tissue, and end effector 300 automatically retracts an unclamped position), as intended, then, at step 512, an emergency recovery mode 530 is not executed.

However, if, at step 510, surgical instrument 100 fails to operate normally or as intended, then, at step 520, an inquiry is executed related to a functionality of the input elements discussed above. If, at step 520, it is determined that surgical instrument 100 has not malfunctioned in a manner which prohibits intended user actuation of the input elements (i.e., broken button, stuck down fire button, broken hall effect sensor, etc.), then, at step 522, it is concluded that end effector 300 is clamped onto underlying tissue and nonresponsive, requiring further intervention.

If, at step 520, it is determined that surgical instrument 100 has malfunctioned in a manner which prohibits intended user actuation of the input elements, then, at step 524, the surgeon enters emergency recovery mode 530.

Emergency recovery mode 530 includes at least the following steps. At step 532, the surgeon removes battery 156 from lower housing portion of handle housing 102 of surgical instrument 100. Then, at step 534, re-insert battery 156 into lower housing portion of handle housing 102 of surgical instrument 100. After re-insertion of battery 156 into surgical instrument 100, at step 536, surgical instrument 100 undergoes a re-boot or boot-up sequence.

Since end effector 300 and adapter 200 are still attached or connected to surgical instrument 100, during the re-boot or boot-up sequence, at step 536, surgical instrument 100 detects the presence of end effector 300 and enters an emergency retraction mode 540.

This simple removal and insertion of battery 156 by the surgeon, and its automatic undergoing of a re-boot or boot-up sequence, may be considered an "intuitive activation." The "intuitive activation" to enter the emergency retraction mode 540 may be achieved even with a maximum degree of damage to surgical instrument (i.e., all input elements damaged and only battery connections, main circuit board 150 and the drive train of surgical system 10 are functioning).

While in the emergency retraction mode 540, at step 542, instrument 100 automatically fully retracts drive assembly 360 of end effector 300 by setting function selection module 163 to operate an appropriate one of drive connectors 118, 120, 122 (i.e., first drive connector 118 which is associated with the opening and closing of tool assembly 304 of end effector 300), and activating second motor 166 in reverse. Drive assembly 360 of end effector 300 is retracted until tool assembly 304 of end effector 300 is returned to a fully open position. With tool assembly 304 of end effector 300 in a fully open position, any tissue trapped within tool assembly 304 may be removed.

Then, either prior to, simultaneously with, or immediately following removal of the trapped tissue from tool assembly 304 of end effector 300, at step 544, all remaining operations or functions of surgical instrument 100 (i.e., including clamping and/or firing) are disabled. Then, at step 546, surgical instrument 100, adapter 200 and/or end effector 300 is/are removed from the surgical site (i.e., withdrawn from the trocar or the like).

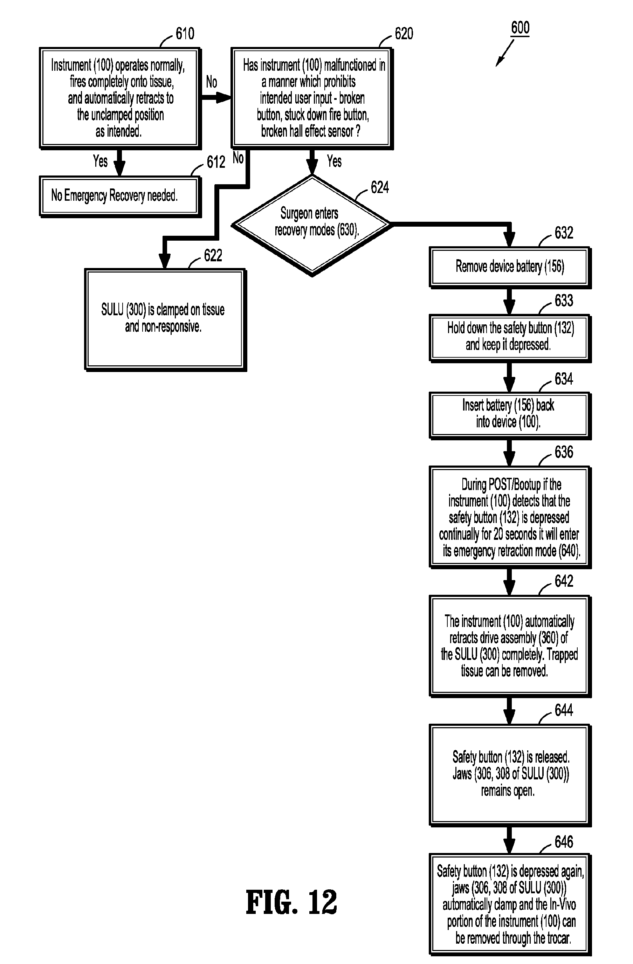

As seen in FIG. 12, a flow chart of an algorithm, in accordance with a second aspect of the present disclosure, is generally designated as 600. Algorithm 600 may be retained locally in circuit board 150 or circuit board 154 of surgical instrument 100, in a memory device (not shown) of end effector 300, or in a remote computer system (not shown) which is in wired or wireless communication with surgical instrument 100 and/or surgical system 10.

In use, during operation of surgical system 10, sensors and the like monitor the operation of surgical instrument 100 and/or end effector 300. As seen in the flow chart of FIG. 12, if, at step 610, surgical instrument 100 operates normally (i.e., end effector 300 fires completely into tissue, and end effector 300 automatically retracts an unclamped position), as intended, then, at step 612, an emergency recovery mode 630 is not executed.

However, if, at step 610, surgical instrument 100 fails to operate normally or as intended, then, at step 620, an inquiry is executed related to a functionality of the input elements discussed above. If, at step 620, it is determined that surgical instrument 100 has not malfunctioned in a manner which prohibits intended user actuation of the input elements (i.e., broken button, stuck down fire button, broken hall effect sensor, etc.), then, at step 622, it is concluded that end effector 300 is clamped onto underlying tissue and nonresponsive.

If, at step 620, it is determined that surgical instrument 100 has malfunctioned in a manner which prohibits intended user actuation of the input elements, then, at step 624, the surgeon enters emergency recovery mode 630.

Emergency recovery mode 630 includes at least the following steps. At step 632, the surgeon removes battery 156 from lower housing portion of handle housing 102 of surgical instrument 100. Then, at step 633, the surgeon depresses and holds down safety button 132 of surgical instrument 100. With safety button 132 depressed and held down, then, at step 634, the surgeon re-inserts battery 156 into lower housing portion of handle housing 102 of surgical instrument 100. After re-insertion of battery 156 into surgical instrument 100, at step 636, surgical instrument 100 undergoes a re-boot or boot-up sequence.

Since safety button 132 has been continuously depressed or held down (i.e., for between about 0 and 30 seconds, desirably about 20 seconds) while battery 156 is re-inserted, during the re-boot or boot-up sequence, at step 636, surgical instrument 100 detects that safety button 132 has been so depressed or held and enters an emergency retraction mode 640.

While in the emergency retraction mode 640, at step 642, instrument 100 automatically fully retracts drive assembly 360 of end effector 300 by setting function selection module 163 to operate an appropriate one of drive connectors 118, 120, 122 (i.e., first drive connector 118 which is associated with the opening and closing of tool assembly 304 of end effector 300), and activating second motor 166 in reverse. Drive assembly 360 of end effector 300 is retracted until tool assembly 304 of end effector 300 is returned to a fully open position. With tool assembly 304 of end effector 300 in a fully open position, any tissue trapped within tool assembly 304 may be removed.

Then, either prior to, simultaneously with, or immediately following removal of the trapped tissue from tool assembly 304 of end effector 300, at step 644, safety button 132 is released, and jaws 306, 308 of end effector 300 remain open.

Then, at step 646, safety button 132 is re-depressed or re-held down, jaws 306, 308 of end effector 300 are automatically re-actuated to a closed or clamped position, to re-clamp onto the tissue, whereby the "in vivo" or re-clamped tissue may be removed from the surgical site (i.e., withdrawn from the trocar or the like).

In the present aspect of the disclosure, the user or surgeon controls the activation of the emergency retraction mode 640 by depressing and holing down safety button 132 for a specified period of time. In accordance with the present disclosure, in an alternate arrangement or set-up, input elements (i.e., control buttons 124, 126; rocker devices 128, 130; etc.) other than safety button 132 may be depressed and held down for a specified period of time during the re-boot or boot-up sequence in order for surgical system 10 and/or surgical instrument 100 to enter the emergency retraction mode 640.

Additionally, in accordance with the present aspect of the disclosure, functionality of surgical system 10 and/or surgical instrument 100, when surgical system 10 and/or surgical instrument 100 is in the emergency retraction mode 640, is limited solely to the closure of jaws 306, 308 of end effector 300.

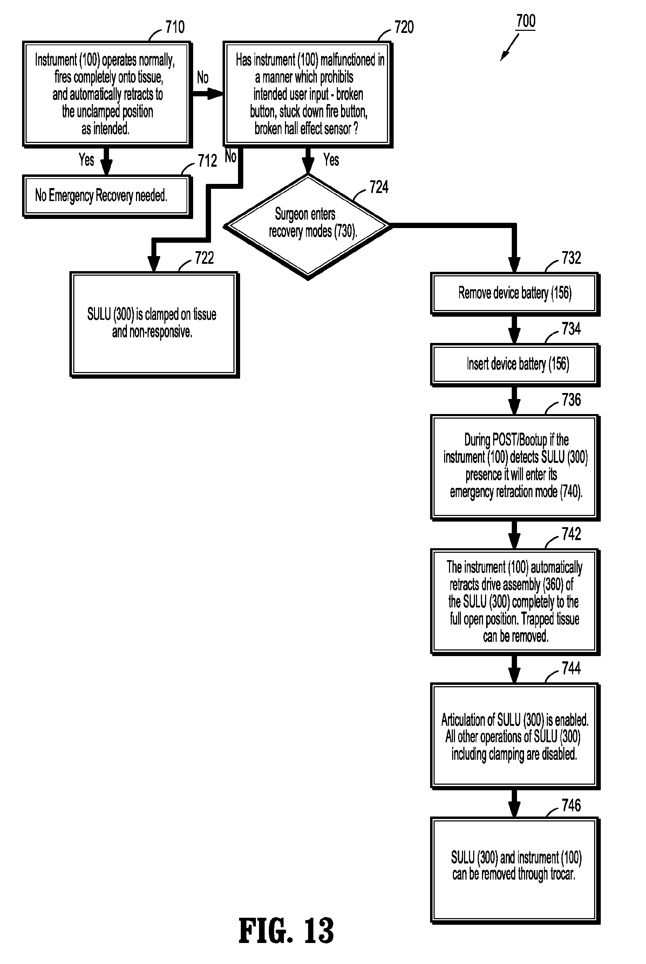

As seen in FIG. 13, a flow chart of an algorithm, in accordance with yet another aspect of the present disclosure, is generally designated as 700. Algorithm 700 may be retained locally in circuit board 150 or circuit board 154 of surgical instrument 100, in a memory device (not shown) of end effector 300, or in a remote computer system (not shown) which is in wired or wireless communication with surgical instrument 100 and/or surgical system 10.

In use, during operation of surgical system 10, sensors and the like monitor the operation of surgical instrument 100 and/or end effector 300. As seen in the flow chart of FIG. 13, if, at step 710, surgical instrument 100 operates normally (i.e., end effector 300 fires completely into tissue, and end effector 300 automatically retracts an unclamped position), as intended, then, at step 712, an emergency recovery mode 730 is not executed.

However, if, at step 710, surgical instrument 100 fails to operate normally or as intended, then, at step 720, an inquiry is executed related to a functionality of the input elements discussed above. If, at step 720, it is determined that surgical instrument 100 has not malfunctioned in a manner which prohibits intended user actuation of the input elements (i.e., broken button, stuck down fire button, broken hall effect sensor, etc.), then, at step 722, it is concluded that end effector is clamped onto underlying tissue and nonresponsive.

If, at step 720, it is determined that surgical instrument 100 has malfunctioned in a manner which prohibits intended user actuation of the input elements, then, at step 724, the surgeon enters emergency recovery mode 730.

Emergency recovery mode 730 includes at least the following steps. At step 732, the surgeon removes battery 156 from lower housing portion of handle housing 102 of surgical instrument 100. Then, at step 734, re-insert battery 156 into lower housing portion of handle housing 102 of surgical instrument 100. After re-insertion of battery 156 into surgical instrument 100, at step 736, surgical instrument 100 undergoes a re-boot or boot-up sequence.

Since end effector 300 and adapter 200 are still attached or connected to surgical instrument 100, during the re-boot or boot-up sequence, at step 736, surgical instrument 100 detects the presence of end effector 300 and enters an emergency retraction mode 740.

This simple removal and insertion of battery 156 by the surgeon, and its automatic undergoing of a re-boot or boot-up sequence, may be considered an "intuitive activation." The "intuitive activation" to enter the emergency retraction mode 740 may be achieved even with a maximum degree of damage to surgical instrument (i.e., all input elements damaged and only battery connections, main circuit board 150 and the drive train of surgical system 10 are functioning).

While in the emergency retraction mode 740, at step 742, instrument 100 automatically fully retracts drive assembly 360 of end effector 300 by setting function selection module 163 to operate an appropriate one of drive connectors 118, 120, 122 (i.e., first drive connector 118 which is associated with the opening and closing of tool assembly 304 of end effector 300), and activating second motor 166 in reverse. Drive assembly 360 of end effector 300 is retracted until tool assembly 304 of end effector 300 is returned to a fully open position. With tool assembly 304 of end effector 300 in a fully open position, any tissue trapped within tool assembly 304 may be removed.

Then, either prior to, simultaneously with, or immediately following removal of the trapped tissue from tool assembly 304 of end effector 300, at step 744, all operations or functions of surgical instrument 100 (i.e., including clamping and/or firing) are disabled, except for an articulation function of end effector 300. Then, at step 746, surgical instrument 100, adapter 200 and/or end effector 300 is/are removed from the surgical site (i.e., withdrawn from the trocar or the like).

It will be understood that various modifications may be made to the embodiments of the presently disclosed adapter assemblies. Therefore, the above description should not be construed as limiting, but merely as exemplifications of embodiments. Those skilled in the art will envision other modifications within the scope and spirit of the present disclosure.

It should be further understood that the foregoing description is only illustrative of the present disclosure. Various alternatives and modifications can be devised by those skilled in the art without departing from the disclosure. Accordingly, the present disclosure is intended to embrace all such alternatives, modifications and variances. The embodiments described with reference to the attached drawing figures are presented only to demonstrate certain examples of the disclosure. Other elements, steps, methods and techniques that are insubstantially different from those described above and/or in the appended claims are also intended to be within the scope of the disclosure.

The invention may be described by reference to the following numbered paragraphs:-
1. An electromechanical surgical system, comprising:
   a hand-held surgical instrument including:
      a handle housing;
      a motor disposed within the handle housing;
      a controller disposed within the handle housing and being in electrical communication with the motor;
      a battery selectively, removably insertable into the handle housing and being in electrical communication with at least one of the motor and the controller when disposed within the handle housing; and
      at least one input element supported on the handle housing and actuatable by a user to send control signals to the controller to operate the motor;
   an end effector selectively and removably connectable to the surgical instrument, the end effector including:
      a jaw assembly having a staple cartridge containing a plurality of staples and an anvil to form the plurality of staples upon firing; and
      a drive assembly at least partially located within the jaw assembly and operatively connectable to the motor when the end effector is connected to the surgical instrument for actuation by the motor;
   wherein the controller is configured to enter an emergency retraction mode when the at least one input element is incapable of providing control signals to the controller to operate the motor, wherein the emergency retraction mode activates the motor to withdraw the drive assembly from any advanced position thereof.
2. The electromechanical surgical system according to paragraph 1, wherein the emergency retraction mode is entered upon a removal and a re-insertion of the battery into the handle housing.
3. The electromechanical surgical system according to paragraph 2, wherein during the emergency retraction mode, the controller undergoes a re-boot, whereby the controller detects that the end effector is connected to the surgical instrument.
4. The electromechanical surgical system according to paragraph 3, wherein in the emergency retraction mode, the drive assembly is automatically retracted.
5. The electromechanical surgical system according to paragraph 4, wherein retraction of the drive assembly to a fully retracted position, opens the jaw assembly.
6. The electromechanical surgical system according to paragraph 4, wherein in the emergency retraction mode the controller disables all remaining functions of the surgical system.
7. The electromechanical surgical system according to paragraph 1, wherein the emergency retraction mode is entered upon a removal of the battery from the handle housing, then a pressing and holding of a safety button, and then a re-insertion of the battery into the handle housing, wherein the safety button is supported on the handle housing.
8. The electromechanical surgical system according to paragraph 7, wherein during the emergency retraction mode, the controller undergoes a re-boot, whereby the controller detects that the end effector is connected to the surgical instrument.
9. The electromechanical surgical system according to paragraph 8, wherein in the emergency retraction mode, the drive assembly is automatically retracted.
10. The electromechanical surgical system according to paragraph 9, wherein retraction of the drive assembly to a fully retracted position, opens the jaw assembly.
11. The electromechanical surgical system according to paragraph 9, wherein, following retraction of the drive assembly, when the safety button is released, the controller does not automatically re-advance the drive assembly.
12. The electromechanical surgical system according to paragraph 11, wherein following a release of the safety button, and following a re-pressing and holding of the safety button, in the emergency retraction mode, the controller activates the motor to re-close the jaw assembly.
13. The electromechanical surgical system according to paragraph 4, wherein in the emergency retraction mode the controller disables all remaining functions of the surgical system with the exception of an articulation function.
14. The electromechanical surgical system according to paragraph 13, wherein in the emergency retraction mode, the surgical instrument is operable to articulate the end effector.
15. The electromechanical surgical system according to paragraph 1, wherein during the emergency retraction mode, the controller undergoes a re-boot.
16. The electromechanical surgical system according to paragraph 15, wherein the controller detects when the end effector is connected to the surgical instrument.
17. The electromechanical surgical system according to paragraph 16, wherein the emergency retraction mode is entered upon a removal and a re-insertion of the battery into the handle housing.
18. The electromechanical surgical system according to paragraph 17, wherein in the emergency retraction mode, the drive assembly is automatically retracted.
19. The electromechanical surgical system according to paragraph 16, wherein the emergency retraction mode is entered upon a removal of the battery from the handle housing, then a pressing and holding of a safety button, and then a re-insertion of the battery into the handle housing.
20. The electromechanical surgical system according to paragraph 19, wherein the safety button is supported on the handle housing.

## Claims

1. An electromechanical surgical system, comprising:
a hand-held surgical instrument including:
a handle housing;
a motor disposed within the handle housing;
a controller disposed within the handle housing and being in electrical communication with the motor;
a battery selectively, removably insertable into the handle housing and being in electrical communication with at least one of the motor and the controller when disposed within the handle housing; and
at least one input element supported on the handle housing and actuatable by a user to send control signals to the controller to operate the motor;
an end effector selectively and removably connectable to the surgical instrument, the end effector including:
a jaw assembly having a staple cartridge containing a plurality of staples and an anvil to form the plurality of staples upon firing; and
a drive assembly at least partially located within the jaw assembly and operatively connectable to the motor when the end effector is connected to the surgical instrument for actuation by the motor;
wherein the controller is configured to enter an emergency retraction mode when the at least one input element is incapable of providing control signals to the controller to operate the motor, wherein the emergency retraction mode activates the motor to withdraw the drive assembly from any advanced position thereof.

2. The electromechanical surgical system according to claim 1, wherein the emergency retraction mode is entered upon a removal and a re-insertion of the battery into the handle housing; preferably wherein during the emergency retraction mode, the controller undergoes a re-boot, whereby the controller detects that the end effector is connected to the surgical instrument; preferably still wherein in the emergency retraction mode, the drive assembly is automatically retracted.

3. The electromechanical surgical system according to claim 2, wherein retraction of the drive assembly to a fully retracted position, opens the jaw assembly; and/or wherein in the emergency retraction mode the controller disables all remaining functions of the surgical system.

4. The electromechanical surgical system according to any preceding claim, wherein the emergency retraction mode is entered upon a removal of the battery from the handle housing, then a pressing and holding of a safety button, and then a re-insertion of the battery into the handle housing, wherein the safety button is supported on the handle housing.

5. The electromechanical surgical system according to claim 4, wherein during the emergency retraction mode, the controller undergoes a re-boot, whereby the controller detects that the end effector is connected to the surgical instrument; preferably wherein in the emergency retraction mode, the drive assembly is automatically retracted.

6. The electromechanical surgical system according to claim 5, wherein retraction of the drive assembly to a fully retracted position, opens the jaw assembly; preferably wherein, following retraction of the drive assembly, when the safety button is released, the controller does not automatically re-advance the drive assembly.

7. The electromechanical surgical system according to claim 6, wherein following a release of the safety button, and following a re-pressing and holding of the safety button, in the emergency retraction mode, the controller activates the motor to re-close the jaw assembly.

8. The electromechanical surgical system according to any preceding claim, wherein in the emergency retraction mode the controller disables all remaining functions of the surgical system with the exception of an articulation function.

9. The electromechanical surgical system according to claim 8, wherein in the emergency retraction mode, the surgical instrument is operable to articulate the end effector.

10. The electromechanical surgical system according to any preceding claim, wherein during the emergency retraction mode, the controller undergoes a re-boot.

11. The electromechanical surgical system according to claim 10, wherein the controller detects when the end effector is connected to the surgical instrument.

12. The electromechanical surgical system according to claim 11, wherein the emergency retraction mode is entered upon a removal and a re-insertion of the battery into the handle housing.

13. The electromechanical surgical system according to claim 12, wherein in the emergency retraction mode, the drive assembly is automatically retracted.

14. The electromechanical surgical system according to claim 13, wherein the emergency retraction mode is entered upon a removal of the battery from the handle housing, then a pressing and holding of a safety button, and then a re-insertion of the battery into the handle housing.

15. The electromechanical surgical system according to claim 14, wherein the safety button is supported on the handle housing.
